# EUROPEAN PATENT APPLICATION

(11) **EP 4 787 390 A1**
(43) Date of publication of application: **05.08.2026**
(21) Application number: 25216942.0
(22) Date of filing: 19.11.2025
(51) Int. Cl.: G21G 1/00

(54) **METHOD FOR PREPARING A RADIOPHARMACEUTICAL AND RADIUM-224 RADIOPHARMACEUTICAL**

(30) Priority: 29.01.2025 JP 2025012529
(71) Applicant: Nippon Nuclear Fuel Development Co., Ltd., Higashi-ibaraki-gun, Ibaraki 311-1313 (JP); Kyoto University, Kyoto-shi, Kyoto 606-8501 (JP)
(72) Inventor: FUKASAWA, Tetsuo, Ibaraki, 3111313 (JP); KITSUNAI, Yuji, Ibaraki, 3111313 (JP); HIGUCHI, Toru, Ibaraki, 3111313 (JP); KANEKO, Masaaki, Ibaraki, 3111313 (JP); YAMAMURA, Tomoo, Kyoto, 6068501 (JP); FUTATSUKAWA, Shoji, Saitama, 3320001 (JP)
(74) Representative: Strehl & Partner mbB

(57) **Abstract**

To provide a method for preparing a radiopharmaceutical that can relatively easily prepare radium-224, a progeny (daughter) nuclide, from thorium-232, which exists in relatively large amounts in nature. A method for preparing a radiopharmaceutical, comprising separating and recovering and purifying the progeny (daughter) nuclide radium-224 from thorium-232 by chemical operation, and using it as a targeted α-ray therapeutic agent.

## Description

### Technical Field

The present invention relates to a radiopharmaceutical used in targeted nuclear medicine (α-ray) treatment and a method for preparing the same, and more particularly, to a technique effective for application to a radiopharmaceutical using radium-224 as an α-ray source.

### Background of Invention

Targeted nuclear medicine (α-ray) treatment (hereafter also referred to as "targeted α-ray treatment") using radiopharmaceuticals that contain nuclides that emit alpha rays (α-rays) in drugs that selectively accumulate in cancer cells is expected as a new cancer treatment method having both high therapeutic effects and low side effects. In particular, since German scientists cured metastatic prostate cancer in 2016 with radiopharmaceuticals using actinium-225 (half-life 10.0 days) as an α-ray source, targeted α-ray treatment has attracted worldwide attention, and many researchers have been conducting related research and development. However, most α-nuclides are difficult to obtain, and relatively easily available nuclides are desired.

As a background technique in this technical field, for example, there is a technique such as Patent Document 1. Patent Document 1 discloses "a preparation relating to a combination of a) radium-224 (²²⁴Ra) and/or progeny (daughter nuclides) of ²²⁴Ra, and b) a DNA repair inhibitor for use in the treatment of cancer or inflammation."

In addition, Non-Patent Document 1 discloses an overview of targeted α-ray treatment and its effectiveness.

Furthermore, Non-Patent Document 2 discloses the preparation relating to a radiopharmaceutical containing lead 212 that emits beta (β) rays.

### Prior Art Document

### Patent Document

Patent Document 1: JP-P2023-541635A (WO2022/058338A1)

### Non-Patent Document

Non-Patent Document 1: Young-Seung Kim, et al., "An overview of targeted alpha therapy", Tumour Biol., Vol.33, 2012 June, p.573-5901
Non-Patent Document 2: Sara Westrom, et al., "Preparation of 212Pb-labeled monoclonal antibody using a novel 224Ra-based generator solution", Nuclear Medicine and Biology, Vol.51, 2017, p.1-9
   2

### Summary of Invention

### Issue to Resolve

Actinium-225 is obtained by milking from thorium-229, a daughter nuclide of uranium-233 produced by countries that were developing nuclear weapons. Since only a limited number of countries possess uranium-233 and thorium-229, research and development to produce actinium-225 in accelerators and reactors is being conducted in countries around the world as an alternative technology. In this case, radium-226 and thorium-230 are used as raw material nuclides, and the low abundance of these nuclides limits the mass production of actinium-225.

As a method for producing actinium-225, there is a method using a nuclear spallation reaction of thorium-232 which exists in nature, but an accelerator for irradiating protons is required.

α-nuclides for medical use other than actinium-225 are known as astatine-211 (half-life 7.22 hours) and radium-223 (half-life 11.4 days), but they are also produced using accelerators or as progeny (daughter) nuclides of the nuclear weapons nuclide uranium-235, and have the same issues as actinium-225. The raw material for astatine-211 is stable nuclide bismuth-209 which exists in nature, but an accelerator is required to irradiate helium nuclei (alpha particles), and because of its short half-life, treatment must be carried out near the manufacturing site.

On the other hand, radium-224, a progeny (daughter) nuclide of thorium-232, has an appropriate half-life of 3.66 days, allowing it to be transported to remote locations, and it can be produced from thorium-232, which is easily available even if it is not a nuclear weapon state, without using large-scale equipment such as accelerators or nuclear reactors.

The above-mentioned Patent Document 1 relates to a combination of radium-224 as a radiopharmaceutical used for cancer treatment and various medicines, and there is no description of a method for preparing (producing) radium-224.

In addition, the above-mentioned Non-Patent Document 1 is a paper summarizing the overview of the targeted α-ray treatment, but there is no description of radium-224. Furthermore, the above-mentioned Non-Patent Document 2 is a paper on radiopharmaceuticals containing lead-212 that emits beta (β) rays, and lead-212 is produced from thorium-228, which is the parent nuclide of radium-224, but there is no description of using radium-224 as a radiopharmaceutical, and there is no description of a method for preparing (producing) thorium-228, which does not exist in nature.

In any of the above-mentioned documents, the preparation (production) of radium-224 is an issue.

Therefore, an objective of the present invention is to provide a method for preparing a radiopharmaceutical that can relatively easily prepare radium-224, a progeny (daughter) nuclide, from thorium-232, which exists in relatively large amounts in nature, and to provide a radium-224 radiopharmaceutical using the same.

### Solution to Problem

To solve the problems, the present invention provides a method for preparing a radiopharmaceutical, comprising separating and recovering and purifying the progeny (daughter) nuclide radium-224 from thorium-232 by chemical operation, and using it as a targeted α-ray therapeutic agent.

The present invention also provides a radium-224 radiopharmaceutical prepared by the above-described method for preparing a radiopharmaceutical.

### Advantageous Effect

According to the present invention, it is possible to realize a method for preparing a radiopharmaceutical that can relatively easily prepare radium-224, a progeny (daughter) nuclide, from thorium-232, which exists in relatively large amounts in nature, and radium-224 radiopharmaceutical using the same.

This will contribute to targeted nuclear medicine (α-ray) treatment using radiopharmaceuticals.

Objects, configurations, and effects other than those described above will be clarified by the description of the following embodiments.

### Brief Description of Drawings

[FIG. 1] FIG. 1 is a flowchart showing a three-stage milking method of the present invention.
[FIG. 2] FIG. 2 is a diagram schematically showing a method for preparing radium-224 according to the first embodiment of the present invention.
[FIG. 3] FIG. 3 is a diagram schematically showing a method for preparing radium-224 according to the second embodiment of the present invention.
[FIG. 4] FIG. 4 is a diagram showing the relationship between the elapsed time and the mass and the radioactivity of each nuclide at each stage in FIG. 1.

### Description of Embodiments

Hereinafter, embodiments of the present invention will be described with reference to the drawings. In the drawings, the same configurations are denoted by the same reference numerals, and a detailed description of repeated parts will be omitted.

### First Embodiment

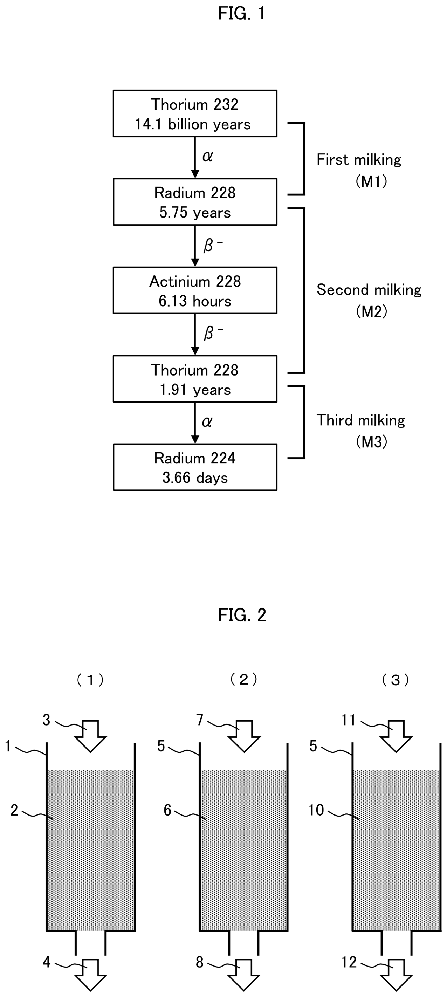

A method for preparing radiopharmaceuticals and radiopharmaceuticals containing radium-224 using the same according to the first embodiment of the present invention will be described with reference to FIGS. 1, 2 and 4.

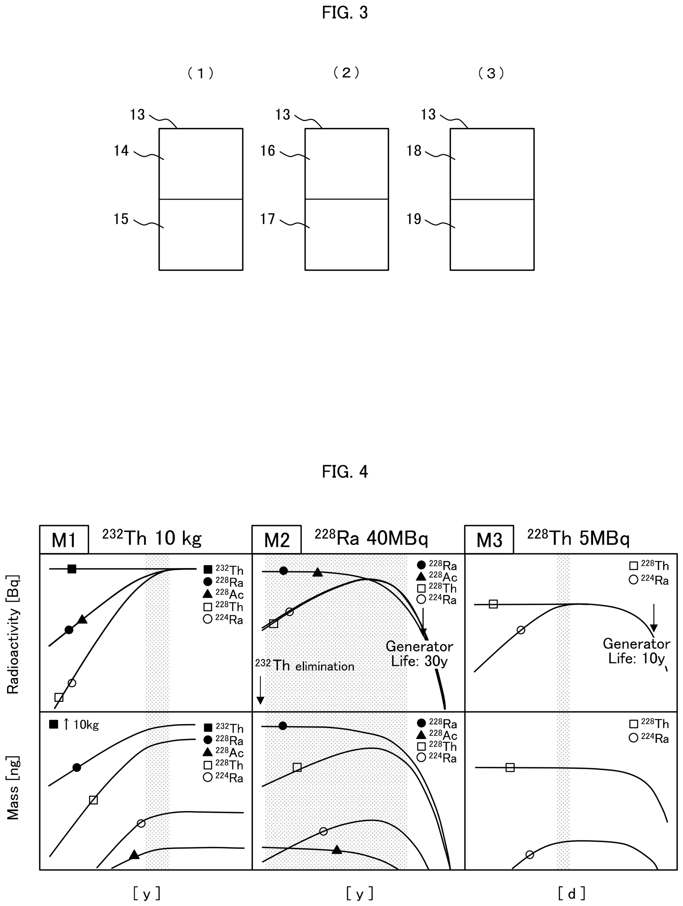

FIG. 1 is a flowchart showing a three-stage milking method of the present invention. FIG. 2 is a diagram schematically showing a method for preparing radium-224 by column chromatography according to the present invention. FIG. 4 is a diagram showing the relationship between the elapsed time and the mass and the radioactivity of each nuclide at each stage in FIG. 1, and it shows the horizontal axis (time) and the vertical axis (mass and radioactivity) as relative values, respectively. In this embodiment, the preparation of radium-224 will be described, which is realized by column chromatography that performed an extraction chromatography method, a leaching method, an ion exchange method, a solvent extraction method, a precipitation method, an adsorption method in a column.

As shown in FIG. 1, in the present invention, radium-224 is recovered from thorium-232, which has been confirmed to exist in an amount of 2 million tons or more on the earth, by a three-stage milking process. "Milking" is an operation of repeatedly separating and extracting short-lived daughter nuclides from parent nuclides having long half-lives when a radiation equilibrium is established.

First, in the first stage (first) milking M1, radium-228, which has a half-life of 5.75 years, is milked from thorium-232, which has a half-life of 14.1 billion years. That is, radium-228 is separated and recovered from thorium-232. In the case of thorium-232 that has not undergone daughter nuclide separation operations for a long period of time, the daughter nuclide radium-228 is in radiation equilibrium, and approximately 40 MBq of radium-228 exists in 10 kg of thorium-232. Further, after radium-228 is separated and recovered from 10 kg of thorium-232, approximately 20 MBq of radium-228 will grow after 5.75 years, approximately 30 MBq of radium-228 will grow after 11.5 years, and approximately 35 MBq of radium-228 will grow after 23 years, respectively.

In this way, even after the radium-228 is recovered once, Radium-228 can be repeatedly recovered every few years to every several decades. If a plurality of thorium-232 is prepared, the recovery interval can be shortened. For example, if 10 units (10 kg/unit) of thorium-232 are prepared and radium-228 is recovered in turn, in the above case, approximately 20 MBq of radium-228 can be recovered approximately every seven months.

Next, in the second stage (second) milking M2, radium-228, which was separated and recovered from thorium-232 in the first stage, is used as the parent nuclide, and via the daughter nuclide actinium-228, which has a half-life of 6.13 hours, the granddaughter (daughter) nuclide thorium-228, which has a half-life of 1.91 years, is recovered. In the second milking M2, the half-lives of radium-228 and thorium-228 are close to each other, so that radiation equilibrium is not reached, and thorium-228 grows as radium-228 decays. From 40 MBq of radium-228, approximately 5 MBq of thorium-228 will grow in about 5 months, approximately 10 MBq of thorium-228 will grow in about 1 year, and approximately 20 MBq of thorium-228 will grow in about 5 years. The growth of thorium-228 reaches its maximum in 2 to 8 years, then decays, and after 30 years, it becomes approximately 1 MBq.

When thorium-228 is recovered in 5 months, the amount of thorium-228 recovered gradually decreases because radium-228 has not decayed much, but it is possible to repeatedly recover near the above-described amount of thorium-228. Further, when thorium-228 is recovered every 5 years, radium-228 decays by about half every 5 years, and approximately 10 MBq of thorium-228 can be recovered the second time, and approximately 5 MBq of thorium-228 can be recovered the third time. Finally (about 30 years later), when radium-228 decays to about 1 MBq or less, radium-228 is supplied by the first milking M1.

Next, in the third stage (third) milking M3, the target nuclide, radium-224, which has a half-life of 3.66 days, is separated and recovered from thorium-228, which has a half-life of 1.91 years, which has been separated and recovered from radium-228. Since the half-life of thorium-228 is more than two orders of magnitude longer than that of radium-224, and the half-life of radium-224 is shorter, thorium-228 after the separation of radium 224 reaches radiation equilibrium with radium-224 after about 5 days or more. That is, radium-224 with the same level of becquerel number as thorium-228 can be recovered every 5 to 10 days until about 10 years when thorium-228 decays. If the amount of thorium-228 recovered in the second milking M2 is 5 MBq, then 5 MBq or a radioactive amount close to 5 MBq of radium-224 can be recovered from the 5 MBq of thorium-228 every few days to every 10 days or so.

Through the above process, radium-224, which is used as a targeted α-ray therapeutic agent, can be separated and recovered from thorium-232, which exists in relatively large quantities in nature, using only chemical operations, without using large equipment such as an accelerator or a nuclear reactor.

FIG. 2 shows a process for realizing three-stage milking by filling or forming a solid phase containing thorium, which is hardly (or slightly) soluble and highly adsorbable, in a column, and then eluting only radium, which is relatively soluble and does not easily form a solid phase.

First, in step (1), an anion exchange resin 2 that has adsorbed thorium-232 (half-life: 14.1 billion years) containing progeny (daughter) nuclides such as radium-228 (half-life: 5.75 years) is loaded into a first column 1, and a radium eluate 4 containing radium-228 and other nuclides is eluted with a radium eluent 3 such as nitric acid having a concentration of about several moles/liter. Thorium-232 remains adsorbed, and all progeny (daughter) nuclides except thorium-228 (half-life 1.91 years) are eluted. This completely separates the radium-228 from the thorium-232.

Next, in step (2), after storing the solution containing the eluted radium-228 and the like for several days or more, it is poured into a second column 5 filled with an anion exchange resin 6, and a radium eluent 7 such as nitric acid is passed through. Thorium-228 grown from radium-228 is adsorbed onto the anion exchange resin 6, while other nuclides 8 such as radium-228 are eluted. This allows radium-228 to be completely removed from thorium-228.

Finally, in step (3), after radium-228 is eluted and removed from thorium-228 adsorbed in step (2), the second column 5 is left as is for one day or more to allow radium-224 to grow, and then a radium eluent 11 such as nitric acid is passed through the second column 5. This causes the target radium-224 (half-life 3.66 days) 12 grown from thorium-228 to elute.

Instead of the anion exchange resin 2 or the anion exchange resin 6, a substance (solid phase) that adsorbs thorium other than the anion exchange resin may be used.

Also, in step (3), the anion exchange resin 6 after removing radium-228 may be transferred to another column, in which case another column (third column) is required.

By using the above-described column chromatography, radium-224, which is used as a targeted α-ray therapeutic agent, can be separated and recovered from thorium-232, which exists in relatively large quantities in nature, using only chemical operations, without using large equipment such as an accelerator or a nuclear reactor.

In step (1), other than the anion exchange resin 2, a substance that adsorbs thorium-232, such as UTEVA (registered trademark) resin, can also be used. Further, thorium 232 loaded into the first column 1 may be in the form of thorium dioxide ThO₂. Thorium dioxide ThO₂ is stable and does not dissolve in nitric acid, but progeny (daughter) nuclides such as radium-228 are dissolved and discharged from the first column 1.

Further, in step (2), in addition to the anion exchange resin 6, any substance that adsorbs or extracts thorium-228, such as UTEVA resin, or any type of solid phase (solid) containing thorium-228 can be used as the substance impregnated with the extracting agent. Furthermore, the nitric acid solution of radium-228 may be stored for several years and then denitrified to form an oxide, or the nitric acid solution of radium-228 may be denitrified immediately after separation and stored for several years, and then loaded into the second column 5 that is not filled with the anion exchange resin 6. Thorium dioxide ThO₂ is stable and does not dissolve in nitric acid, but radium-228 and the like are dissolved and discharged from the second column 5.

If one day or more has passed since radium-228 has been completely removed, in step (3), the radium eluent 11 such as nitric acid is passed through, whereby the target radium-224 grown from thorium-228 can be dissolved and recovered from the second column 5.

By storing the first column 1 of step (1) for a long period of time (several years or more), the progeny (daughter) nuclides grow, so that radium-224 can be repeatedly obtained by the above-described procedure.

According to the present embodiment described above, thorium-232, which exists in large quantities in nature (a reserve amount of 2 million tons or more), can be utilized, and radium-224, which is effective as an α-nuclide for radiopharmaceuticals, can be prepared without using large equipment such as an accelerator or a nuclear reactor.

### Second Embodiment

A method for preparing radiopharmaceuticals and radiopharmaceuticals containing radium-224 using the same according to the second embodiment of the present invention will be described with reference to FIG. 3.

FIG. 3 is a diagram schematically showing the preparation of radium-224 by the solvent extraction method according to the present invention. In this embodiment, a process for realizing three-stage milking by a solvent extraction method will be described.

First, in step (1), thorium-232 containing a progeny (daughter) nuclide such as radium-228 is dissolved in nitric acid or the like, and put into a solvent extraction device 13 to form an aqueous phase 15. To this, a solvent (organic phase) 14 such as TBP (TributylPhosphate) that extracts thorium is added and shaken, only thorium is extracted and transferred from the aqueous phase 15 to an organic phase 14. If necessary, extraction with a fresh solvent is repeated to completely transfer thorium-232 in the aqueous phase 15 to the organic phase 14.

Next, in step (2), the organic phase 14 containing thorium-232 is removed from the solvent extraction device 13, and an aqueous phase 17 containing radium-228 is stored for several days or more, and then fresh solvent (organic phase) 16 such as TBP is added and shaken. Thorium-228 grown from radium-228 is extracted and transferred from the aqueous phase 17 to an organic phase 16. If necessary, extraction with a fresh solvent is repeated to transfer thorium-228 as much as possible into the organic phase 16 while preventing radium-228 from being mixed into the organic phase 16.

Finally, in step (3), the aqueous phase 17 containing radium-228 is removed from the solvent extraction device 13, and an organic phase 18 containing thorium-228 is stored for about one day or more, and fresh solution (aqueous phase) 19 such as nitric acid is added and shaken. Radium-224 grown from thorium-228 is back extracted and transferred from the organic phase 18 to an aqueous phase 19.

By using the above-described solvent extraction operation, radium-224, which is used as a targeted α-ray therapeutic agent, can be separated and recovered from thorium-232, which exists in relatively large quantities in nature, using only chemical operations, without using large equipment such as an accelerator or a nuclear reactor.

Note that as the solvent extraction device 13 in the second embodiment, a separatory funnel, a mixer settler, a centrifugal extractor, or the like can be used, and not only single-stage extraction but also multi-stage extraction may be performed. Further, a continuous (multi-stage) solvent extraction apparatus such as a pulse column may also be used.

According to the above-described embodiments of the present invention, radium-224 can be prepared (manufactured) from thorium-232 existing in nature by milking in three stages without using an accelerator or a nuclear reactor.

The α-nuclides used in targeted α-ray treatment have half-lives ranging from several hours to 10 days or so, and are prepared (manufactured) from raw material nuclides using nuclear reactions. In addition, if the raw material nuclide does not exist in nature, it will have to be prepared in some method. Most of the α-nuclides used in targeted α-ray treatment, which have been known so far, are derived from nuclides that do not exist in nature, and even nuclides that do exist in nature are produced using large equipment such as accelerators or nuclear reactors. Therefore, there was the problem for the procurement of raw material nuclides, and cancer patients had to receive treatment in a place close in time to a large equipment.

According to the present invention, the above-mentioned problems can be solved because thorium-232 is used as a raw material nuclide, which is abundant in nature, and the α-nuclide can be prepared using a laboratory-scale equipment. In particular, once thorium-228 that does not contain radium-228 is prepared and the second column 5 that adsorbed thorium-228 is taken to a hospital where radiopharmaceuticals can be synthesized, cancer patients can be treated for about 10 years without supplementing with radium-224.

The invention is not limited to the embodiments described above, and includes various modifications. For example, the embodiments described above have been described in detail to facilitate understanding of the invention, and the invention is not necessarily limited to those including all the configurations described above. A part of a configuration of a certain embodiment can be replaced with a configuration of another embodiment, and the configuration of another embodiment can be added to the configuration of a certain embodiment. A part of a configuration according to each embodiment may be added to, deleted from, or replaced with another configuration.

### Industrial Applicability

The present invention has potential applications in the medical field of cancer treatment using radiopharmaceuticals containing α-nuclides, in the radiochemistry field of separating progeny (daughter) nuclides from natural thorium, in the nuclear reprocessing field of removing impurities from irradiated thorium fuels, and in the waste treatment field of separating nuclides from radioactive waste.

### Reference Sign List

1: First column
2, 6, 10: Anion exchange resin
3, 7, 11: Radium eluent
4: Radium eluate
5: Second column
8: Other nuclides such as radium-228
12: Radium-224
13: Solvent extraction device
14, 16, 18: Organic phase
15, 17, 19: Aqueous phase

## Claims

1. A method for preparing a radiopharmaceutical,
comprising separating and recovering and purifying the progeny (daughter) nuclide radium-224 from thorium-232 by chemical operation, and using it as a targeted α-ray therapeutic agent.

2. The method for preparing a radiopharmaceutical according to claim 1,
wherein the chemical operation includes at least one of a leaching method, an ion exchange method, a solvent extraction method, an extraction chromatography method, a precipitation method, and an adsorption method.

3. The method for preparing a radiopharmaceutical according to claim 1, comprising the steps of:
(a) a step of recovering radium-228 from thorium-232 by milking,
(b) a step of recovering thorium-228 from the radium-228 by milking,
(c) a step of recovering radium-224 from the thorium-228 by milking.

4. The method for preparing a radiopharmaceutical according to claim 3, further comprising the steps of:
(d) after the step (c), a step of waiting for six years or more for the growth of the radium-228 (half-life 5.75 years) from the thorium-232 (half-life 14.1 billion years),
(e) a step of waiting for 4 days or more for the growth of the thorium-228 (half-life 1.91 years) from the radium-228,
(f) a step of waiting for one day or more for the growth of the radium-224 (half-life 3.66 days) from the thorium-228.

5. The method for preparing a radiopharmaceutical according to claim 1,
Wherein the thorium-228 recovered from the thorium-232 by two-stage milking is used as the parent nuclide, and radium-224 is recovered from the thorium-228 by the third stage milking.

6. The method for preparing a radiopharmaceutical according to claim 1, comprising the steps of:
(g) a step of filling an oxide of thorium-232 into a first column and passing a radium eluent through it to recover the coexisting radium-228,
(h) a step of waiting for thorium-228 to grow from the recovered radium-228, separating the thorium-228 from the radium-228, converting the thorium-228 into an oxide, and then filling the thorium-228 into a second column,
(i) a step of using the thorium-228 as the parent nuclide of radium-224, the radium eluent is passed through the second column every 1 to 10 days to recover the radium-224.

7. The method for preparing a radiopharmaceutical according to claim 1, comprising the steps of:
(j) a step of filling a first anion exchange resin that has adsorbed thorium-232 containing progeny (daughter) nuclide into a first column, and passing a radium eluent through the first column to recover the coexisting radium-228,
(k) a step of waiting for thorium-228 to grow from the recovered radium-228, injecting the thorium-228 into a second column filled with a second anion exchange resin, and passing the radium eluent through the second column to elute and separate the radium-228,
(l) a step of using the thorium-228 obtained after the separation of the radium-228 as the parent nuclide of the radium-224, and passing the radium eluent through every 1 to 10 days to recover the radium-224.

8. The method for preparing a radiopharmaceutical according to any one of claims 1 to 7,
wherein the radium-224 recovered from the thorium-232 is loaded into an agent that accumulates in a specific cancer cell.

9. A radium-224 radiopharmaceutical prepared by the method for preparing a radiopharmaceutical according to any one of claims 1 to 7.
